Europäisches Patentamt

European Patent Office

Office européen des brevets

(11) Veröffentlichungsnummer: **0 125 459**
**A2**

(12) ## EUROPÄISCHE PATENTANMELDUNG

(21) Anmeldenummer: **84103740.1**

(22) Anmeldetag: **05.04.84**

(51) Int. Cl.³: **A 61 B 3/06**

(30) Priorität: **19.04.83 DE 3314171**

(43) Veröffentlichungstag der Anmeldung:
**21.11.84 Patentblatt 84/47**

(84) Benannte Vertragsstaaten:
**DE FR GB NL**

(71) Anmelder: **Oculus Optikgeräte GmbH**

**D-6330 Wetzlar 17(DE)**

(72) Erfinder: **Bolle, Franz, Dr.med.**
**Königskoppel 16**
**D-2370 Rendsburg(DE)**

(74) Vertreter: **Missling, Arne, Dipl.-Ing. et al,**
**Patentanwälte Dipl.-Ing. R. Schlee Dipl.-Ing. A. Missling**
**Bismarckstrasse 43**
**D-6300 Giessen(DE)**

(54) Anomaloskop.

(57) Anomaloskope dienen zur Untersuchung des menschlichen Farbensinns und haben eine optische Einrichtung für die Erzeugung einer Rot-, einer Grün- und einer Gelbspektralfarbe, eine Einrichtung für die Mischung der spektralen Farben grün und rot derart, daß bei einer Verstärkung einer Spektralfarbe die jeweils andere entsprechend verringert wird. Desweiteren sind Anzeigemittel für die Intensität der spektralen Farben und für die Anteile der grünen und roten Spektralfarben für die Ermittlung des Anomalquotienten vorhanden. Um ein derartiges Anomaloskop technisch einfach im Aufbau mit kleinen Abmessungen zu gestalten, wobei die Messung des menschlichen Farbensinns nach der Rayleigh-Gleichung ausgewertet werden kann, ist als Lichtquelle für die roten und grünen Spektralfarben eine Zweifarben-Lumineszenzdiode und für die gelbe Spektralfarbe eine Einfarben-Lumineszenzdiode vorgesehen, die jeweils einen engen und differenzierten Spektralbereich aufweisen. Die Dioden werden so nahe wie möglich nebeneinander angeordnet. Vorteilhaft wird zur Einengung des Spektralbereichs vor jeder Lumineszenzdiode ein Interferenzfilter angeordnet.

Fig. 2

Patentanwälte

Dipl.-Ing. Richard Schlee
Dipl.-Ing. Arne Missling

C300 Lahn-Giessen 1
Bismarckstrasse 43
Telefon: (0641) 71019

0125459

Mi/V 15.188

OCULUS Optikgeräte GmbH
6330 Wetzlar 17

## Anomaloskop

Die Erfindung betrifft ein Anomaloskop zur Untersuchung des menschlichen Farbensinns mit einer optischen Einrichtung für die Erzeugung einer Rot-, einer Grün- und einer Gelbspektralfarbe, einer Einrichtung für die Mischung der spektralen Farben grün und rot, derart, daß bei einer Verstärkung einer Spektralfarbe die jeweils andere Spektralfarbe entsprechend verringert wird und mit Anzeigemitteln für die Intensität der spektralen Farben und für die Anteile der grünen und roten Spektralfarben für die Ermittlung des Anomalquotienten nach der Rayleigh-Gleichung.

Die Prüfung des menschlichen Farbensinns hat Bedeutung im Bereich der Medizin, für den Befähigungsnachweis von Menschen, die in bestimmten Bereichen des Verkehrs oder der Technik arbeitsmäßig eingesetzt werden sollen, insbesondere dann, wenn es auf das Erkennen farbiger optischer Signale ankommt. Die Prüfbarkeit des Farbunterscheidungsvermögens ist in der Praxis besonders wichtig im Rot-Grün-Bereich.

-2-

Die Prüfungen des menschlichen Farbensinns werden heute meist mit Pigmentproben (z.B. sogenannten pseudoisokromatischen Tafeln), mit durchscheinenden Farbfiltergläsern (Farbtestscheibe) oder durch Mischung und Vergleichen spektraler Lichter mit dem Anomaloskop nach Nagel durchgeführt.

Beim Anomaloskop nach Nagel wird die Farbsichtigkeit in bezug auf das Rot-Grün-Sehen bestimmt. Die Ermittlung erfolgt durch den Vergleich eines binären Gemisches aus einem roten und einem grünen Spektrallicht mit einem monokromatischen gelb. Aus dem Mischungsverhältnis und der eingestellten Leuchtdichte des Vergleichsgelbs kann die Farbennormalsichtigkeit bzw. Art und Grad der Farbenfehlsichtigkeit des Prüflings in bezug auf das Rot-Grün-Sehen erkannt werden. Das Anomaloskop nach Nagel hat eine mechanisch sehr aufwendige Konstruktion. Die Rot-Grün-Mischung wird durch zwei parallel liegende Eintrittsspalten bewirkt, deren äußere Spaltbacken unveränderlich feststehen und auf die rote bzw. grüne Wellenlänge eingestellt sind. Die inneren Kanten dieser Spalte werden von einer gemeinsamen, mittels Feinschraube verschiebbaren Spaltbacke gebildet, durch deren Verschiebung der eine Spalt um den gleichen Betrag erweitert wie der andere verengt wird und damit das Mischungsverhältnis der beiden Spektrallichter verändert werden kann. Neben diesen beiden Mischspalten ist ein dritter, unilateraler Spalt für das Vergleichslicht angeordnet, der auf die gelbe Farbe eingestellt ist und mittels einer beweglichen Spaltbacke den Gelbspalt nach der langwelligen Seite hin öffnet. Die Schrauben für die Einstellung der Spalte haben Skalen. Bei geschlossenem Spalt zeigt die Skala O und bei voll geöffnetem Spalt den Wert 87 an. Für die beiden Mischspalten beträgt die nutzbare Breite 73 Intervalle, bei der Stellung O ist der Rotspalt geschlossen und der Grünspalt voll geöffnet, während bei der Stellung 73 der Grünspalt geschlossen und der Rotspalt voll geöffnet ist. Das erzeugte Licht gelangt über eine aufwendige Optik in das Auge des Beobachters, so daß dieser in der unteren Hälfte des Gesichtsfeldes das homogene gelbe und in der oberen Hälfte je nach Stellung der Mischspalte ein homogenes rotes oder ein grünes Spektrallicht oder eine Mischung dieser Lichter sieht. Die Einstellung am Anomaloskop sowie dessen Justierung erfolgt gemäß der DIN-Norm 6160. Ein Anomaloskop nach Nagel ist jedoch ausgesprochen aufwendig und teuer.

Es ist auch bereits vorgeschlagen, Lumineszenzdioden für Farbteste heranzuziehen, wobei zwei gleichfarbige Dioden verwendet werden, deren Helligkeiten moduliert werden. Moduliert man die Lichter der Wellenlängen der beiden Lumineszenzdioden gegensinnig, d.h. läßt man das eine aufblinken, während sich das andere verdunkelt, und umgekehrt, so muß die Intensität der Lichter entsprechend gewählt werden, damit derjenige kein Flimmern mehr wahrnimmt, der in diesem Spektralbereich nichts wahrnimmt. Das Einsatzgebiet ist jedoch sehr beschränkt, da mit dieser Methode keine Differenzierung zwischen Anomalie und Anopie festgestellt werden kann. Im übrigen gestattet dieses Gerät nicht die übliche Differenzierung nach der Rayleigh-Gleichung, d.h. die Feststellung des Anomalquotienten.

Was die Prüfungen mit Pigmentproben und Farbfiltergläsern betrifft, so zeigen diese Methoden eine hohe Ungenauigkeit und somit nicht immer brauchbare Ergebnisse.

Der Erfindung liegt die Aufgabe zugrunde, ein Anomaloskop der eingangs genannten Art so auszubilden, daß dieses technisch einfach im Aufbau und somit kostengünstig herzustellen ist, kleine Abmessungen besitzt und die Messung des menschlichen Farbensinns und seine Auswertung nach der Rayleigh-Gleichung gestattet.

Diese Aufgabe wird mit den Merkmalen des kennzeichnenden Teils des Anspruches 1 gelöst.

Gemäß der Erfindung werden als Lichtquelle für die Rot-Grün-Spektralfarbe sowie für die gelbe Spektralfarbe Lumineszenzdioden gewählt, wobei für die Rot-Grün-Spektralfarbe eine Zweifarben-Lumineszenzdiode eingesetzt wird. Diese beiden Lumineszenzdioden werden räumlich sehr eng nebeneinander angeordnet, so daß das resultierende Mischlicht gleichzeitig mit dem Licht der gelb aussendenden Luminiszenzdiode auf Farbgleichheit und Helligkeit verglichen werden kann. Das Mischungsverhältnis rot-grün bzw. die Leuchtdichte der gelbemitierenden Lumineszenzdiode kann an Skalen abgelesen werden.

Die Einstellung des farbmeßtechnischen Normalbeobachters gilt als Normaleinstellung. Die Einstellung des Anomaloskops soll so vorgenommen werden, daß für die Gleichung des Normalbeobachters die Meßschraube auf den Wert 40 zeigt. Das Strahlendichteverhältnis im Mischlicht beträgt dann für diese Einstellung rot/grün = 22,2. Die Gelbschraube soll bei der Mittelgleichung etwa auf den Wert 15 zeigen. Der Anomalquotient errechnet sich aus der Einstellung der Rot-Grün-Skala. Das Testen des Probanden erfolgt entsprechend dem Anomaloskop nach Nagel. Gemäß einem weiteren Vorschlag der Erfindung ist im Gerät ein Mikrocomputer eingesetzt, der die Skaleneinstellungen ermittelt und auf einer weiteren Anzeige direkt den Anomalquotienten anzeigt. Vorteilhaft werden die Lumineszenzdioden über eine spannungsstabilisierte Stromquelle gespeist, damit nicht zufällige Spannungsschwankungen zu Meßfehlern führen.

Der Untersuchungsvorgang kann durch den Einsatz eines Mikrocomputers gefördert werden. Die Steuerung kann derart erfolgen, daß bei Beginn der Untersuchung zunächst ein Extrembereich geschaltet wird, z.B. ein Mischbereich, wobei zunächst ein bestimmter Rotwert gezeigt wird und der Gelbwert variiert wird. Der zu Untersuchende wird dann aufgefordert, mit Hilfe der Helligkeitsveränderung des Gelblichtes eine Gleichstellung der beiden Dioden zu erzielen. Erreicht man hier keine Gleichstellung, so liegt für diesen Bereich keine Farbsinnstörung vor.

Anschließend wird ein weiterer Extrembereich untersucht, indem vom Mikrocomputer ein bestimmter Grünwert vorgegeben wird und mit Hilfe der Helligkeitsveränderung des Gelbwertes eine Gleichstellung einzustellen versucht wird. Kann die Gleichstellung in diesem Farbbereich erzielt werden, so erfolgt als nächste Prüfung der Mischbereich rot/grün-Diode, wobei der Untersuchte beide Testhälften auf gleiche Werte einzustellen hat. Der Farbentüchtige wird exakt einen Wert einstellen, der in einem gewissen Toleranzbereich immer wieder reproduzierbar ist.

Die automatische Bedienerführung erleichtert und verkürzt den Untersuchungsvorgang.

Je nach Anforderungen, die an das Gerät gestellt werden, kann vor den Dioden noch zusätzlich ein Interferenzfilter angeordnet werden, so daß der Spektralbereich der Lumineszenzdioden sehr eng eingegrenzt werden kann.

Die Steuerung des Diodenstroms und damit der Leuchtdichte kann analog über die Gleichstromverstärkung von Kleinsignaltransistoren im Kollektorkreis durch Veränderung des Basisstroms am Basisspannungsteiler mittels Potentiometer erfolgen. Es ist jedoch denkbar, die Dioden mit einem konstanten Diodenstrom alternierend über einen astabilen Multivibrator zu versorgen, der ein variables Tastverhältnis mit einer Frequenz hat, die höher liegt als das Auflösungsvermögen des menschlichen Auges (z.B. 100 Hertz). Die Mischung der Lichter erfolgt über Änderung des Tastverhältnisses rot-grün mit einem Potentiometer. Die Einschaltzeit der jeweiligen Lumineszenzdiode bestimmt die vom Auge wahrgenommene Farbe und damit die Art der Mischfarbe. Die Helligkeit der gelben Lumineszenzdiode wird in gleicher Weise gepulst durch Verschiebung des Tastverhältnisses gelb - aus.

Das Digitalgerät kann wegen seiner geringen Stromaufnahme von einer 9-Volt-Batterie gespeist werden. Das Anomaloskop kann in einer Größe von 10 x 6 x 3 cm oder auch kleiner gefertigt werden.

Der Vorteil des erfindungsgemäß ausgebildeten Anomaloskops besteht in seiner Einfachheit und in seinem niedrigen Herstellungspreis, in seiner genauen Steuerbarkeit und in seiner mechanischen Unempfindlichkeit. Hinzu kommen seine kleinen Abmessungen und die Möglichkeit, die von den Lumineszenzdioden erzeugten Lichter ohne weitere optische Hilfsmittel und unabhängig von zusätzlichen Lichtquellen zu betrachten.

Ein Ausführungsbeispiel der Erfindung ist im folgenden anhand der Zeichnung näher beschrieben, in dieser zeigen:

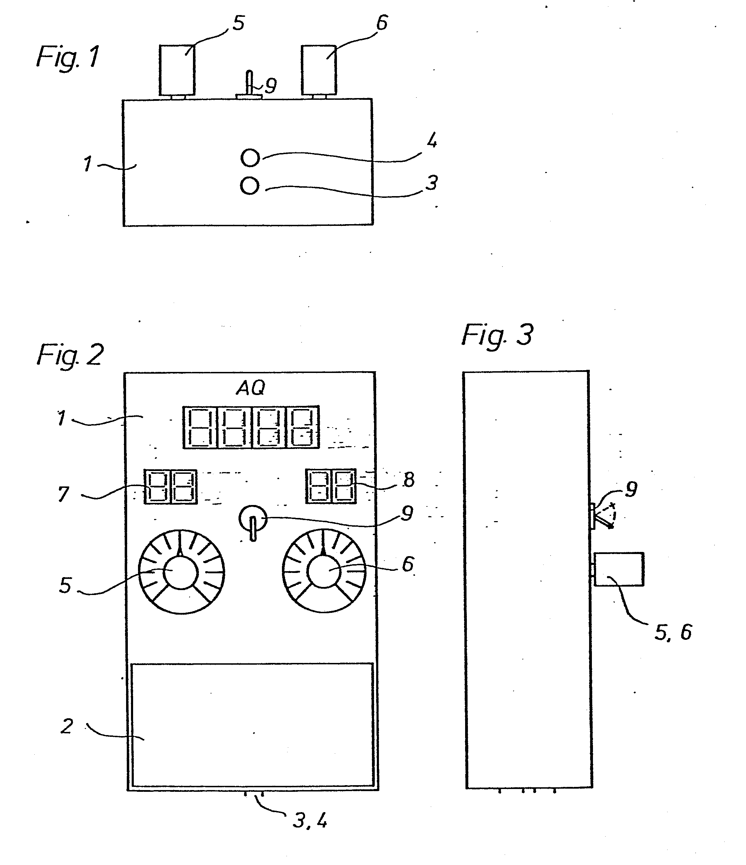

Fig. 1 - 3     eine Seitenansicht, eine Stirnansicht und eine Draufsicht auf ein erfindungsgemäß ausgebildetes Anomaloskop und

Fig. 4     ein Prinzipschaltbild des in den Fig. 1 - 3 dargestellten Anomaloskops.

Das Anomaloskop ist in den Figuren mit 1 bezeichnet. Das Anomaloskop besteht aus einem rechteckigen Gehäuse, das die Stromversorgung 2 aufnimmt, die z.B. aus einer Batterie oder einem Trafo mit Konstanthaltung bestehen kann. An der Stirnseite des Anomaloskops 1 sind zwei Dioden 3, 4 angeordnet, von denen die Diode 3 die gelbe Spektralfarbe und die Diode 4 die grüne und die rote Spektralfarbe aussendet. Mittels zweier Regler 5, 6 wird die Leuchtstärke der Lumineszenzdiode 3 bzw. das Mischungsverhältnis der Zweifarben-Lumineszenzdiode 4 eingestellt. Über den beiden Reglern 5, 6 sind zwei Skalen 7, 8 angeordnet, an denen die eingestellten Werte abgelesen werden können. Ein Schalter 9 dient zum Ein- und Ausschalten des Gerätes.

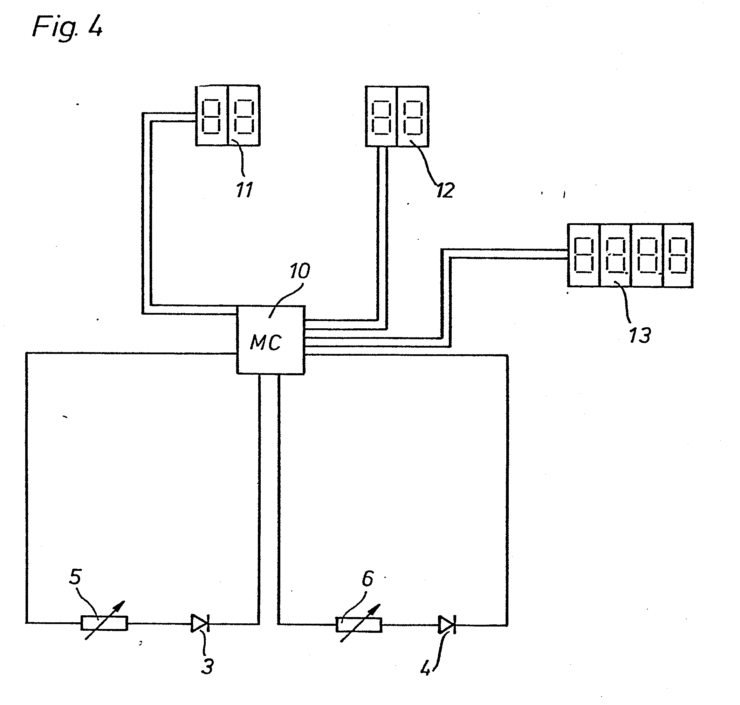

In Fig. 4, die ein Prinzipschaltbild eines Anomaloskops gemäß der Erfindung wiedergibt, wird der durch die Dioden 3 und 4 fließende Strom von einem Mikrocomputer 10 erfaßt und die auf den Reglern 5, 6 eingestellten Werte unmittelbar zusätzlich digital in 11 und 12 angezeigt. Eine weitere Digitalanzeige 13 weist unmittelbar den Anomalquotienten aus, so daß dieser ohne zusätzliche Rechnung unmittelbar abgelesen werden kann. Dieser Anomalquotient wird nach der Rayleigh-Gleichung ermittelt.

Patentansprüche:

1. Anomaloskop zur Untersuchung des menschlichen Farbensinns mit einer optischen Einrichtung für die Erzeugung einer Rot-,einer Grün- und einer Gelbspektralfarbe, einer Einrichtung für die Mischung der spektralen Farben grün und rot derart, daß bei einer Verstärkung einer Spektralfarbe die jeweils andere entsprechend verringert wird und mit Anzeigemitteln für die Intensität der spektralen Farben und für die Anteile der grünen und roten Spektralfarben für die Ermittlung des Anomalquotienten, dadurch gekennzeichnet, daß als Lichtquelle für die roten und grünen Spektralfarben eine Zweifarben-Lumineszenzdiode und für die gelbe Spektralfarbe eine Einfarben-Lumineszenzdiode mit einem jeweils engen und differenzierten Spektralbereich dient und daß die beiden Dioden nebeneinander liegend angeordnet sind.

2. Anomaloskop nach Anspruch 1, dadurch gekennzeichnet, daß vor jeder Lumineszenzdiode ein Interferenzfilter angeordnet ist.

3. Anomaloskop nach Anspruch 1 oder 2, dadurch gekennzeichnet, daß die Lumineszenzdioden über eine spannungsstabilisierte Stromquelle gespeist werden.

4. Anomaloskop nach einem der Ansprüche 1 bis 3, dadurch gekennzeichnet, daß die Anzeigemittel die eingestellten Werte digital anzeigen und daß ein Mikrocomputer vorgesehen ist, der die eingestellten Werte nach der Rayleigh-Gleichung umrechnet und die erhaltenen Anomalquotientenwerte digital anzeigt.

Fig. 1

Fig. 2

Fig. 3

*Fig. 4*